# FASCICULE DE BREVET EUROPEEN

(11) **EP 1 235 603 B1**
(45) Date de publication et mention de la délivrance du brevet: **24.05.2006**
(21) Numéro de dépôt: 00985412.6
(22) Date de dépôt: 07.12.2000
(51) Int. Cl.: A61M 5/32

(54) **ENSEMBLE DE SECURITE POUR UNE SERINGUE**
SICHERHEITSVORRICHTUNG FÜR EINE SPRITZE
SAFETY SYSTEM FOR A SYRINGE

(30) Priorité: 07.12.1999 FR 9915387
(43) Date de publication de la demande: 04.09.2002
(73) Titulaire: Plastef Investissements, 95520 Osny (FR)
(72) Inventeur: CHEVALLIER, Stéphane, F-78420 Carrières sur Seine (FR)
(74) Mandataire: Intes, Didier Gérard André
(86) Numéro de dépôt international: PCT/FR2000/003397
(87) Numéro de publication internationale: WO 2001/041841

(56) Documents cités:
- FR-A- 2 654 346
- FR-A- 2 778 853
- JP-A- 11 319 090
- US-A- 5 342 320

## Description

La présente invention concerne un ensemble d'un dispositif de support de sécurité pour une seringue et d'une seringue ayant un corps présentant une collerette, un piston et une aiguille, le dispositif comprenant un fourreau de support pour le corps de la seringue et des moyens de sécurité qui comprennent un fourreau intérieur, susceptible de coulisser par rapport au fourreau de support entre une position rentrée d'attente dans laquelle il est sensiblement rentré à l'intérieur du fourreau de support en étant apte à se trouver autour d'une partie d'extrémité au moins du corps de la seringue portant l'aiguille et une position sortie de protection dans laquelle il dépasse en dehors du fourreau de support, pour former une protection autour de l'aiguille, le dispositif comportant en outre, des moyens de retenue susceptibles d'être activés pour retenir le fourreau intérieur en position rentrée d'attente dans le fourreau de support et d'être désactivés pour autoriser la sortie du fourreau intérieur sous l'action de moyens de poussée.

Le brevet US 5 492 536 montre un ensemble dans lequel un bouclier de protection est formé par des lamelles élastiques, disposées autour du corps de la seringue et initialement retenues dans une position d'attente par des moyens de retenue détachables. En fin de course, le piston vient détacher ces moyens de retenue et, sous l'effet d'un ressort de poussée, le bouclier s'avance sur le corps de la seringue de telle sorte que ces lamelles viennent se refermer autour de l'aiguille.

Le fonctionnement de ce système n'est assuré que s'il est correctement manipulé, l'utilisateur devant en effet prendre garde à ce que la position de ses doigts sur la seringue ne gêne pas le déplacement du bouclier. De plus, les lamelles risquent de pincer la peau du patient en se refermant autour de l'aiguille.

Le brevet US 5 562 626 montre un système dans lequel le fourreau a la forme d'un tube retenu sur une collerette de la seringue par des pattes élastiques. En fin de course, le piston vient détacher ces pattes de la collerette permettant ainsi la rentrée du corps de la seringue à l'intérieur du corps du fourreau. Cette fois encore, la sécurité n'est garantie que si l'utilisateur manipule ce système avec précaution. En effet, il maintient le système par le fourreau en le pinçant en général entre l'index et le majeur, tandis qu'il manipule le piston avec le pouce. La rentrée du corps de seringue n'est autorisée que si l'utilisateur éloigne son pouce du fourreau d'une distance suffisante pour permettre la rentrée complète de l'aiguille dans le fourreau.

La demande de brevet EP 0 405 039 présente un système du même type, qui présente sensiblement les mêmes inconvénients.

Le document EP 0 966 983 décrit un ensemble d'un dispositif de support de sécurité pour une seringue et d'une seringue dont le corps est supporté dans le fourreau de support de manière à être déplaçable par rapport à ce dernier. C'est ce déplacement qui, en fin d'injection, permet de déclencher la sortie du fourreau intérieur. Pour commander ce déplacement l'opérateur doit, à la fin de l'injection du produit contenu dans la seringue, exercer une forte pression sur cette dernière, ce qui provoque une seconde pénétration de l'aiguille et risque d'être douloureux voire dangereux pour le patient.

L'invention vise à proposer un dispositif de support de sécurité et un ensemble constitué d'un dispositif de ce type et d'une seringue permettant de remédier aux inconvénients précités.

Ce but est atteint avec l'ensemble selon la revendication 1 grâce au fait qu'il comporte des moyens de calage aptes à caler le corps de la seringue par rapport au fourreau de support, ces moyens de calage comprenant une surface de butée et une nervure d'encliquetage, ménagées à l'intérieur du fourreau de support et entre lesquelles la collerette est apte à être calée, et au fait que les moyens de retenue sont susceptibles d'être désactivés en fonction de la position du piston de la seringue, tandis que le corps de la seringue reste fixe dans le fourreau de support

Selon l'invention, le fourreau de support et le fourreau intérieur forment en quelque sorte deux tubes concentriques. Le corps de la seringue est engagé dans ces tubes en étant calé par rapport au fourreau de support qui constitue le tube extérieur, de manière à ce que le corps de la seringue reste fixe dans le fourreau de support Ce dispositif de calage assure que la commande du fourreau intérieur est sure et tout à fait indolore pour le patient L'utilisateur manipule ce dispositif en saisissant le fourreau de support (par exemple en le pinçant entre l'index et le majeur) et en poussant le piston de la seringue pour réaliser l'injection, par exemple avec le pouce. Lorsque le piston de la seringue parvient dans la position voulue (en fin de course), les moyens de retenue du fourreau intérieur dans le fourreau de support sont désactivés permettant ainsi la sortie automatique de ce fourreau intérieur en dehors du fourreau de support. Le déclenchement du fourreau intérieur pour l'amener dans sa position sortie de protection est directement assuré par le piston, lorsque celui-ci parvient en fin de course. En fin d'injection, l'utilisateur n'a à effectuer aucun mouvement supplémentaire pour permettre la sortie du fourreau intérieur, il lui suffit de continuer le geste qu'il avait pour réaliser l'injection, geste habituel du praticien. De plus, il n'a pas à prendre de précautions particulières quant au positionnement de ses doigts sur le fourreau de support et sur le piston pour permettre la sortie du fourreau intérieur.

Selon une disposition particulièrement avantageuse, le fourreau de support comporte une partie d'extrémité arrière et une partie principale, ladite partie arrière présentant des moyens de calage pour la collerette du corps de la seringue et une surface d'appui pour les moyens de poussée.

Avantageusement, le fourreau de support, le fourreau intérieur et les moyens de poussée forment un tout dans lequel le corps de la seringue peut être inséré par une poussée axiale, jusqu'à ce que la collerette dudit corps coopère avec lesdits moyens de calage.

Les moyens de poussée comprennent avantageusement un ressort dont on limitera de préférence la raideur ou la précontrainte à des valeurs suffisamment faibles pour éviter que la sortie du fourreau intérieur ne vienne exercer un appui désagréable sur la peau du patient.

Avantageusement, le dispositif comporte en outre des moyens de blocage aptes à bloquer le fourreau intérieur dans sa position sortie de protection.

Une fois la sortie du fourreau intérieur déclenchée par la désactivation des moyens de retenue, celui-ci vient, sous l'effet des moyens de poussée, adopter la position dans laquelle il est bloqué par les moyens de blocage. On prévient ainsi les risques de rentrée intempestive du fourreau intérieur dans le fourreau de support.

Ces moyens de blocage comprennent avantageusement une surface de blocage ménagée sur la périphérie interne du fourreau de support et au moins un élément de blocage, solidaire du fourreau intérieur et apte à coopérer avec cette surface en position sortie de protection du fourreau intérieur.

Ceci constitue une manière simple pour réaliser les moyens de blocage précédemment évoqués.

Selon une disposition avantageuse, les moyens de retenue comprennent des languettes élastiques et des surfaces de retenue, lesdites languettes ou lesdites surfaces étant solidaires du fourreau intérieur, les languettes étant retenues sur les surfaces de retenue dans leur configuration active de retenue et étant susceptibles d'être déplacées élastiquement vers leur configuration inactive dans laquelle elles échappent auxdites surfaces.

Avantageusement, les languettes élastiques sont orientées sensiblement axialement, sont solidaires du fourreau intérieur et coopèrent dans leur configuration active avec des surfaces de retenue formées sur la périphérie interne du fourreau de support.

Ces languettes axiales sont de préférence disposées de telle sorte qu'une partie d'actionnement du piston (par exemple la tête du piston) vienne à leur contact en fin de course du piston, de manière à les solliciter vers leur configuration inactive.

Les languettes axiales sont avantageusement réalisées en une seule pièce avec l'un ou l'autre des fourreaux intérieur et extérieur.

Selon un mode de réalisation particulièrement avantageux, ces languettes élastiques sont solidaires du fourreau intérieur et constituent également des éléments de blocage aptes à coopérer avec la surface de blocage en position sortie de protection du fourreau intérieur.

Les languettes élastiques réalisées de cette manière servent non seulement à la retenue du fourreau intérieur dans le fourreau de support en position d'attente mais aussi à son blocage en position sortie.

Les fourreaux intérieur et extérieur sont avantageusement réalisés en matière plastique. Selon une disposition avantageuse, au moins une portion du fourreau de support est réalisée en un matériau opaque. Cette précaution permet de masquer aux yeux du patient le mécanisme interne du dispositif du support (moyens de calage, ressort...).

L'invention sera bien comprise et ses avantages apparaîtront mieux à la lecture de la description détaillée qui suit, d'un mode de réalisation représenté à titre d'exemple non limitatif.

La description se réfère aux dessins annexés, sur lesquels :
- la figure 1 est une vue en coupe axiale d'un ensemble d'un dispositif de support de sécurité et d'une seringue conforme à l'invention, en position d'attente avant l'utilisation de la seringue,
- la figure 2 est une vue en coupe axiale selon la ligne II-II de la figure 1,
- la figure 3 est une vue agrandie de la zone III de la figue 2,
- la figure 4 est une vue partielle en perpective prise selon la direction IV indiquée sur la figure 2, et
- la figure 5 est une coupe axiale analogue à celle de la figure 1, mais dans laquelle l'ensemble est représenté après l'utilisation de la seringue, en situation de protection de l'aiguille de cette dernière,
- la figure 6 est une vue analogue à la figure 1 pour une variante de réalisation,
- la figure 7 est une vue en coupe selon la ligne VII-VII de la figure 6, à une échelle agrandie,
- la figure 8 est une vue partielle en perspective, prise selon la flèche VIII de la figure 6.

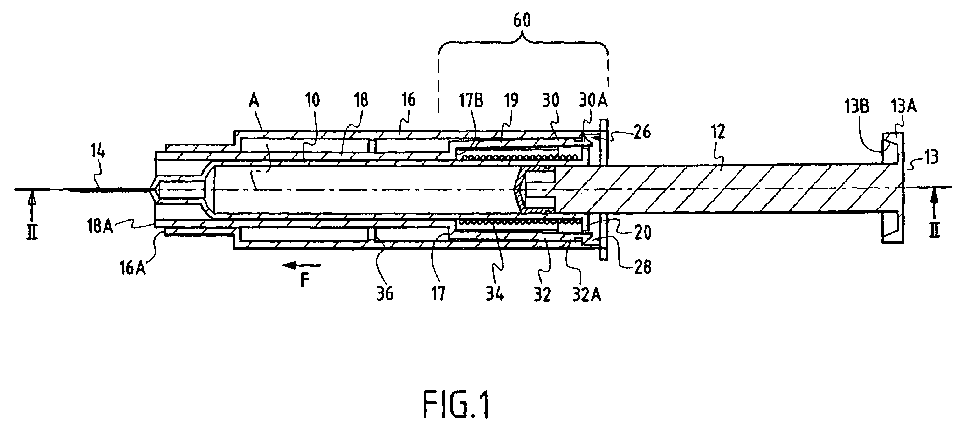

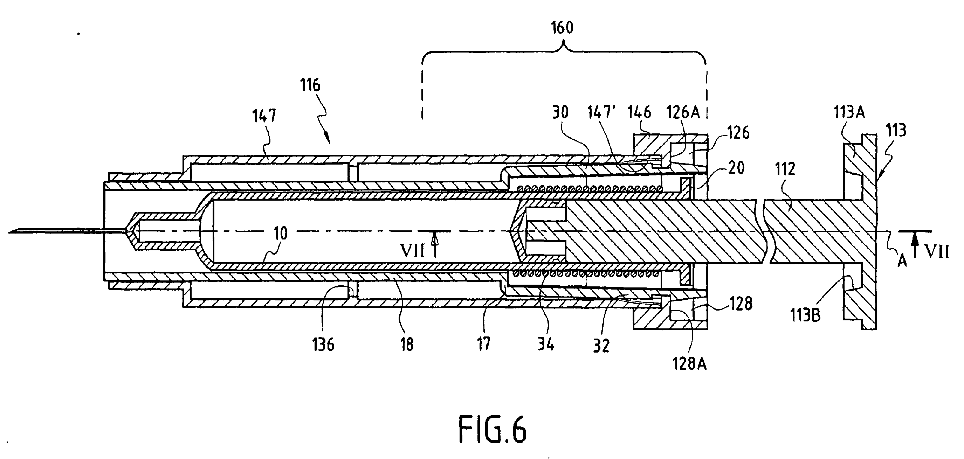

La seringue représentée sur les figures comporte un corps 10 dans lequel peut coulisser un piston 12 entre une position sortie d'attente avant injection représentée sur la figure 1 et une position rentrée après injection représentée sur la figure 5. A l'opposé du piston, une aiguille d'injection 14 est raccordée au corps 10. Classiquement, le corps de la seringue est tubulaire, en verre ou en plastique.

Le dispositif de support de cette seringue comporte un fourreau extérieur de support 16 et un fourreau intérieur 18. En position d'attente avant injection, le fourreau intérieur 18 est globalement rentré dans le fourreau extérieur 16, tandis que le corps de la seringue est engagé dans ledit fourreau intérieur 18 et est calé par rapport au fourreau extérieur 16 de telle sorte que l'aiguille 14 dépasse au-delà des extrémités avant 16A et 18A des fourreaux.

Bien entendu, les extrémités avant sont celles qui sont destinées à se trouver près du point d'injection. Le sens vers l'avant est le sens F de poussée du piston.

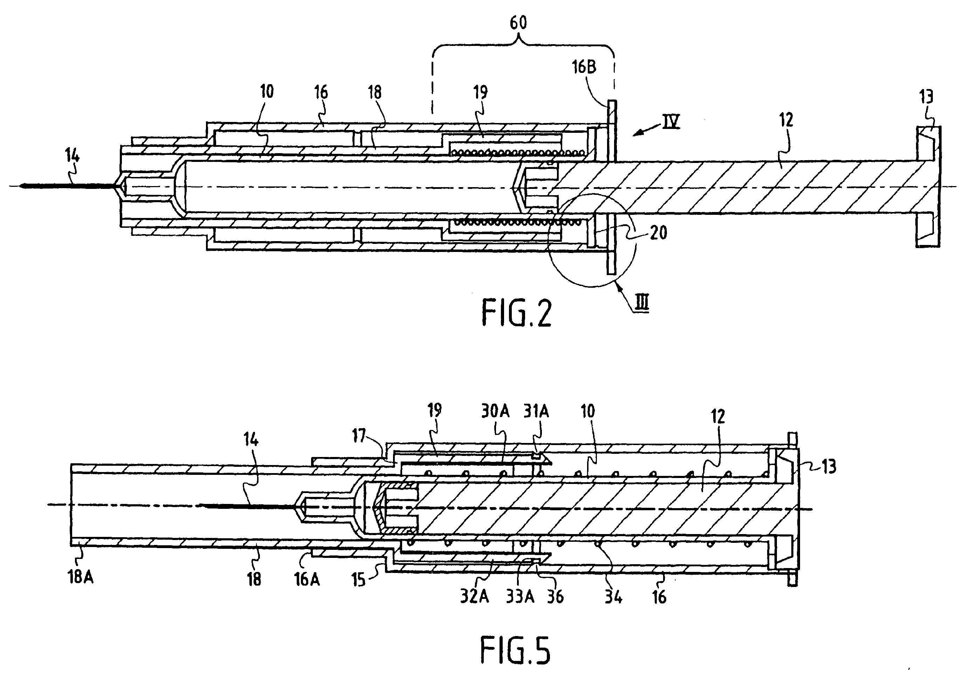

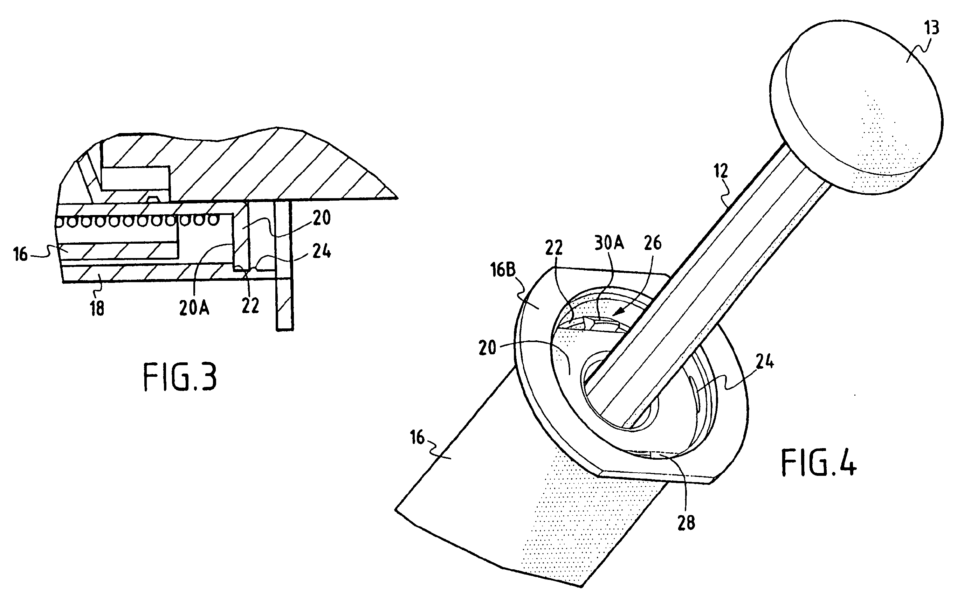

Le corps de la seringue est calé par rapport au fourreau extérieur 16 par des moyens de calage appartenant à ce fourreau, qui coopèrent avec une collerette radiale 20 que présente l'extrémité arrière (opposée à l'aiguille) du corps de la seringue. En effet, comme on le voit mieux sur les figures 2 et 3, la collerette 20 est calée entre une surface de butée 22 et une nervure d'encliquetage 24 ménagées à l'intérieur du fourreau de support 16 (sur sa périphérie interne). La nervure d'encliquetage 24 est formée en arrière de la surface de butée 22 et, lors de l'insertion du corps de la seringue dans le dispositif de support, la paroi du fourreau 16 se déforme légèrement élastiquement pour permettre à la collerette 20 de passer la nervure 24. Par exemple, la surface de butée 22 est formée sur un épaulement circulaire, tandis que la nervure d'encliquetage 24 peut être formée par un bourrelet circulaire continu ou, comme dans l'exemple représenté, par deux arcs de nervure diamétralement opposés. Pour son calage par rapport au fourreau extérieur 16, la collerette 20 coopère avec la périphérie interne de ce fourreau comme on l'a vu précédemment. Toutefois, des passages sont ménagés entre la collerette 20 et la périphérie intérieure du fourreau de support 16.

Ces passages 26 et 28 sont mieux visibles sur les figures 1 et 4. Par exemple, le contour de la collerette est formé selon un cercle de diamètre adapté au calage dans le fourreau 16, cercle coupé selon deux cordes diamétralement opposées pour ménager les passages 26 et 28. Ces passages servent à la retenue du fourreau intérieur 18 dans sa position d'attente.

En effet, comme le montre la figure 1, la partie d'extrémité arrière du fourreau 18 porte des languettes axiales 30 et 32 dont les extrémités libres sont accrochées sur des surfaces de retenue. Avantageusement, comme c'est le cas dans l'exemple représenté, ces surfaces de retenue sont formées sur l'épaulement 22, dans des zones de ce dernier laissées dégagées par les passages 26 et 28. Les nervures d'encliquetage 24 sont quant à elles avantageusement interrompues dans ces régions pour éviter de gêner l'accrochage des extrémités libres des languettes 30 et 32 sur l'épaulement 22.

Les languettes 30 et 32 sont élastiques et, comme le montre la figure 1, elles ont une tendance naturelle à s'écarter légèrement en s'éloignant de l'axe géométrique A du dispositif.

Les languettes élastiques 30 et 32 qui sont représentées sont donc solidaires du fourreau intérieur 18 et s'accrochent sur le fourreau extérieur 16. On pourrait également imaginer que des languettes soient plutôt solidaires de la périphérie interne du fourreau extérieur 16 et s'accrochent sur le fourreau intérieur 18 ou que les languettes élastiques aient un sens d'accrochage inversé par rapport à celui que montre le dessin pour venir s'accrocher sur la collerette 20 du corps de la seringue. Dans toutes ces variantes, ces languettes doivent pouvoir être décrochées lorsque le piston 12 de la seringue parvient dans une position de fin de course.

Les extrémités libres 30A et 32A des languettes 30 et 32 dépassent en effet légèrement dans les passages 26 et 28. De son côté, le piston 12 comporte une tête d'actionnement 13 apte à coopérer avec les extrémités libres 30A et 32A des languettes pour les solliciter dans leur position inactive.

Dans l'exemple représenté, les languettes 30 et 32 sont accrochées sur le fourreau extérieur 16 et leurs extrémités forment des rampes orientées vers l'extérieur. De son côté, la tête 13 présente une portion en forme de bride axiale 13A dirigée vers l'avant dont la périphérie interne 13B est inclinée de telle sorte que, lorsque le piston parvient en fin de course, cette périphérie interne 13B coopère avec les rampes des extrémités des languettes et sollicite ces dernières en les rapprochant de l'axe A. Si l'on utilisait des languettes ménagées sur la périphérie interne du fourreau 16 ou des languettes accrochées sur la collerette 20, on pourrait modifier la conformation de la tête d'actionnement 13 pour que, en fin de course du piston, elle ait tendance à écarter élastiquement les languettes de retenue de l'axe A, de manière à libérer également le fourreau intérieur 18.

Les moyens de poussée favorisent la poussée vers l'avant (dans le sens F indiqué sur la figure 1) du fourreau intérieur 18 lorsque les languettes 30 et 32 sont libérées par la tête du piston. Comme on le voit sur les figures, ces moyens de poussée comprennent avantageusement un ressort 34 qui, lorsque la seringue est mise en place dans les fourreaux, est disposé autour du corps 10 de cette seringue et prend appui sur une première surface d'appui appartenant à l'un des éléments constitués par le corps de la seringue et par le fourreau de support et sur une deuxième surface d'appui appartenant au fourreau intérieur.

Dans l'exemple représenté, la première surface d'appui est formée sur la collerette 20, l'extrémité arrière du ressort 34 reposant sur la face frontale 20A de cette collerette. Dans sa partie arrière, le manchon intérieur 18 présente une portion 19 de diamètre augmenté, les portions 18 et 19 étant raccordées par un épaulement 17. Le ressort est logé dans l'espace annulaire ménagé, dans la portion 19, entre le fourreau intérieur 18 et le corps 10 de la seringue. La deuxième surface d'appui est donc formée par la face arrière 17B de l'épaulement 17. La portion 19 a une forme globalement tubulaire et est fendue sur une partie de sa longueur pour former les languettes élastiques 30 et 32, ces dernières faisant en outre saillie sur l'arrière par rapport au reste de l'extrémité arrière de la portion 19.

Les figures 1 à 4 montrent le dispositif dans la position rentrée d'attente du fourreau intérieur 18 à l'intérieur du fourreau 16. Dans cette position, les languettes de retenue sont accrochées, le ressort 34 est armé, et l'aiguille 14 d'une seringue placée dans le dispositif dépasse au-delà des extrémités avant des fourreaux.

La figure 5 montre en revanche le dispositif après utilisation de la seringue. Dans cette position, le piston 12 est en fin de course, sa tête 13 venant pratiquement en butée contre la collerette 20. La tête 13 a actionné les languettes 30 et 32 qui se sont décrochées de l'épaulement 22. Le ressort 34 a poussé le fourreau intérieur 18 vers l'avant de telle sorte que ce dernier dépasse largement au-delà de l'extrémité avant 16A du fourreau de support 16, sur une longueur telle qu'il forme un "bouclier de protection" tout autour de l'aiguille 14 et sur une longueur suffisante pour éviter les risques de contact d'un utilisateur avec cette dernière.

Dans cette situation, le fourreau intérieur 18 est calé vers l'avant par la venue en butée de son épaulement 17 avec un épaulement 15 formé dans la région avant du fourreau extérieur 16. A l'arrière de cet épaulement, la périphérie interne du fourreau 16 présente un diamètre adapté à recevoir la portion 19 du fourreau 18, tandis que la partie d'extrémité avant 16A du fourreau 16 qui se trouve au-delà de l'épaulement 15 présente un diamètre adapté à celui de la partie du fourreau 18 qui est en avant de l'épaulement 17.

Le dispositif de l'invention comporte des moyens de blocage aptes à bloquer le fourreau intérieur 18 dans cette position de sortie. En d'autres termes, ces moyens de blocage servent à éviter qu'un simple appui sur l'extrémité libre 18A du fourreau 18 ne permette la rentrée de ce fourreau dans le fourreau extérieur 16. Ces moyens de blocage comprennent une surface de blocage ménagée sur la périphérie interne du fourreau de support 16 et au moins un élément de blocage, solidaire du fourreau intérieur 18 et apte à coopérer avec cette surface de blocage en position sortie de protection du fourreau intérieur.

Dans l'exemple représenté, la surface de blocage est simplement formée par un bourrelet annulaire 36 ménagé en relief sur la périphérie interne du fourreau 16. Les languettes élastiques 30 et 32 précédemment évoquées constituent quant à elles également les éléments de blocage qui coopèrent avec cette surface de blocage. Sous les rampes de leurs extrémités libres, les languettes présentent en effet des renfoncements 31A et 33A qui sont ouverts vers l'extérieur. Les faces arrière de ces renfoncements servent à l'accrochage sur l'épaulement 22, tandis que leurs faces avant servent au blocage sur le bourrelet 36.

Lorsqu'une seringue est mise en place dans le dispositif de l'invention, l'utilisateur manipule l'ensemble constitué par ce dispositif et par cette seringue en tenant le fourreau extérieur 16 par exemple entre l'index et le majeur, en plaçant ses doigts sur une collerette 16B formée à l'extrémité arrière de ce fourreau, et en manipulant le piston par le pouce. Lorsque le piston parvient en fin de course, il décroche les languettes 30 et 32A qui émergent par les passages 26 et 28, et le fourreau intérieur est repoussé jusqu'à sa position de sortie, dans laquelle il est bloqué par les languettes 30 et 32 coopérant avec le bourrelet 36.

Le corps de la seringue reste calé par les moyens de calage précédemment évoqués. Il est par ailleurs supporté vis-à-vis d'un éventuel débattement transversal par le contact entre sa périphérie externe cylindrique et la périphérie interne de la partie avant du fourreau 18.

Sur les figures 6 à 8, les éléments inchangés par rapport aux figures 1 à 5 conservent les mêmes références. Cette variante concerne essentiellement la réalisation du fourreau extérieur qui permet une livraison du dispositif de support en situation "armée", le corps de la seringue pouvant être inséré par l'utilisateur sans aucune intervention sur le support en lui-même.

Sur la figure 6, le fourreau de support 116 comprend une partie d'extrémité 146 et une partie principale 147. Cette partie principale 147 forme un corps globalement cylindrique qui s'étend sur pratiquement toute la longueur du fourreau de support. La partie d'extrémité 146, quant à elle, est seulement présente vers l'arrière du fourreau de support (à l'extrémité opposée à l'aiguille 14).

Globalement, cette partie d'extrémité arrière présente la forme d'une rondelle qui est fixée sur l'extrémité libre de la partie principale par tous moyens appropriés (soudure, collage...). Elle présente une nervure interne 121 formant, sur sa face arrière, une surface du butée 122 contre laquelle repose la collerette 20 du corps de la seringue. La rondelle 146 présente également des moyens formant une nervure d'encliquetage 124 analogue à la nervure 24.

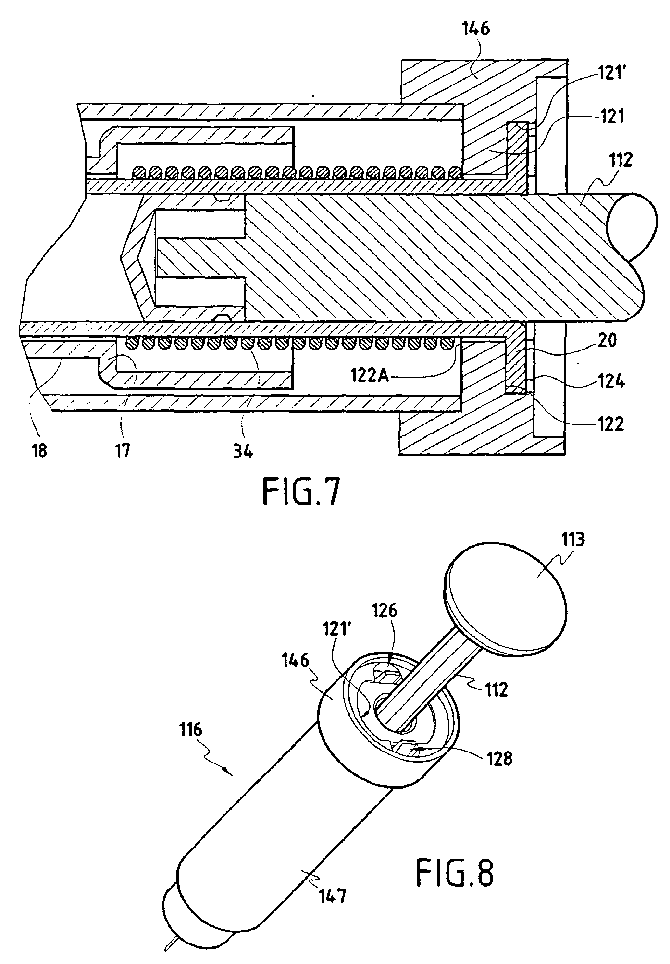

Comme on le comprend en comparant les figures 6 et 7, la nervure interne 121 présente deux portions d'arc qui délimitent entre elles une dimension diamétrale minimale très légèrement supérieure au diamètre extérieur du corps de la seringue. La nervure interne 121 a donc la forme d'un anneau dans lequel sont pratiquées des encoches 126 et 128 qui permettent le passage des languettes élastiques 30, 32 de retenue du fourreau intérieur 18. Comme on le voit mieux sur les figures 7 et 8, la face arrière de la nervure 121 présente une zone en renfoncement 121' adaptée à recevoir la collerette 20 sensiblement à complémentarité de forme pour la caler angulairement.

Ces languettes 30 et 32 sont retenues par rapport à la partie d'extrémité 146. En effet, les fonds des encoches 126 et 128 présentent des surfaces de retenue, respectivement 126A et 128A, avec lesquelles coopèrent des zones d'accrochage des languettes.

Ainsi, les encoches 126 et 128 constituent, pour la variante de la figure 6, l'équivalent des passages 26 et 28 de la variante des figures 1 à 5. Les languettes 30 et 32 dépassent à travers les encoches 126 et 128 en étant retenues par les surfaces de retenue 126A et 128A de manière à pouvoir être sollicitées par la tête du piston, de la même manière que la variante des figures 1 à 5. En effet, la tête 113 présente une portion en forme de bride axiale 113A, dont la périphérie interne présente des rampes 113B aptes à coopérer avec les languettes pour les rapprocher de l'axe A du dispositif.

La nervure interne 121 sert, par sa face frontale 122A, de première surface d'appui pour l'extrémité arrière du ressort.

Grâce à ces dispositions, le dispositif de support constitué par les fourreaux intérieur et extérieur et par le ressort peut constituer un tout qui est vendu avec le ressort armé, c'est-à-dire que le ressort est précontraint entre la surface de retenue 122A précédemment évoquée et l'épaulement 17 du fourreau intérieur, ce dernier étant retenu par ces languettes dans la partie d'extrémité 146 du fourreau de support.

Ce dispositif peut être commercialisé dans cet état aux laboratoires qui peuvent se contenter de disposer à l'intérieur le corps d'une seringue rempli de liquide à injecter, avec un piston classique ou, éventuellement, un piston dont la tête a été modifiée pour présenter la conformation adaptée à solliciter les languettes 30 et 32 de manière à faire sortir le fourreau intérieur. La surface d'appui 122A pour le ressort s'étend sur pratiquement un cercle complet à l'exception des zones (de taille limitée) des encoches 126 et 128. Le ressort est donc parfaitement supporté en position armée et sa périphérie interne définit un canal qui permet l'introduction aisée du corps de la seringue dans le dispositif de support. Pour monter le corps de la seringue dans son dispositif de support, il suffit alors à l'utilisateur (le laboratoire) de pousser axialement le corps de seringue vers l'avant jusqu'à ce que sa collerette 20 vienne s'encliqueter entre la surface de butée 122 et la nervure d'encliquetage 124.

Comme on le voit mieux sur la figure 6, pour permettre que les languettes occupent naturellement leur position écartée sans être gênées par la paroi de la partie principale 147 du fourreau de support, cette dernière présente, au voisinage de son extrémité arrière qui porte la partie d'extrémité 146, une zone de dégagement 147' dans laquelle son diamètre interne est supérieur à son diamètre interne courant.

La périphérie interne de la partie 147 du fourreau 116 présente une nervure 136 analogue à la nervure 36 des figures 1 à 5, servant au blocage du fourreau intérieur 18 en position sortie.

Comme on le voit également sur la figure 6, les surfaces de retenue 126A et 128A pour les languettes 30 et 32 sont légèrement décalées vers l'avant par rapport à la surface de butée 122. Ceci permet de faire en sorte que, pour déverrouiller le fourreau intérieur, il soit nécessaire d'insérer la tête 113 du piston 112 relativement profondément à l'intérieur de la partie d'extrémité 146. Lorsque le dispositif est vendu sans le corps de la seringue, et sans piston, on limite les risques de déclenchement intempestif du fourreau intérieur car il est difficile d'insérer de manière fortuite un outil suffisamment profondément dans les encoches 126 et 128 pour rappeler élastiquement les languettes vers l'intérieur.

Avantageusement, au moins la partie arrière 60 ou 160 du fourreau de support 16 ou 116 est rendu opaque pour éviter que le mécanisme disposé à l'intérieur de ce fourreau ne soit visible par le patient devant subir la piqûre.

## Revendications

1. Ensemble d'un dispositif de support de sécurité pour une seringue et d'une seringue ayant un corps (10) présentant une collerette (20), un piston (12) et une aiguille (14), le dispositif comprenant un fourreau de support (16 ; 116) pour le corps de la seringue et des moyens de sécurité qui comprennent un fourreau intérieur (18), susceptible de coulisser par rapport au fourreau de support entre une position rentrée d'attente dans laquelle le fourreau intérieur (18) est sensiblement rentré à l'intérieur du fourreau de support (16 ; 116) en étant apte à se trouver autour d'une partie d'extrémité au moins du corps de la seringue (10) portant l'aiguille (14) et une position sortie de protection dans laquelle le fourreau intérieur (18) dépasse en dehors du fourreau de support, pour former une protection autour de l'aiguille, le dispositif comportant en outre, des moyens de retenue (30, 32, 22 ; 126A, 128A) susceptibles d'être activés pour retenir le fourreau intérieur (18) en position rentrée d'attente dans le fourreau de support (16 ; 116) et d'être désactivés pour, autoriser la sortie du fourreau intérieur (18) sous l'action de moyens de poussée (34),
**caractérisé en ce qu'**il comporte des moyens de calage (22, 24 ; 122, 124) qui sont solidaires du fourreau de support (16 ; 116) et qui maintiennent le corps (10) de la seringue de manière fixe dans ce demier, ces moyens de calage comprenant une surface de butée (22 ; 122) contre laquelle la collerette (20) est immobilisée par des moyens formant une nervure d'encliquetage (24 ; 124), et **en ce que** les moyens de retenue (30, 32, 22 ; 126A, 128A) sont susceptibles d'être désactivés en fonction de la position du piston (12) de la seringue, tandis que le corps (10) de la seringue reste maintenu fixement dans le fourreau de support (16 ; 116) par immobilisation de la collerette (20) de la seringue maintenue par les moyens de calage (22, 24 ; 122, 124).

2. Ensemble selon la revendication 1, **caractérisé en ce que** le fourreau de support (116) comporte une partie d'extrémité arrière (146) et une partie principale (147), ladite partie arrière présentant des moyens de calage (122, 124) pour la collerette (20) du corps de la seringue (10) et une surface d'appui (122A) pour les moyens de poussée (34).

3. Ensemble selon la revendication 2, **caractérisé en ce que** le fourreau de support (116), le fourreau intérieur (18) et les moyens de poussée (34) forment un tout dans lequel le corps de la seringue (10) peut être inséré par une poussée axiale, jusqu'à ce que la collerette (20) dudit corps coopère avec lesdits moyens de calage (122, 124).

4. Ensemble selon l'une quelconque des revendications 1 à 3, **caractérisé en ce que** les moyens de retenue comprennent des languettes élastiques (30, 32) et des surfaces de retenue (22 ; 126A, 128A), lesdites languettes ou lesdites surfaces étant solidaires du fourreau intérieur (18), les languettes (30, 32) étant retenues sur les surfaces de retenue (22 ; 126A, 128A) dans leur configuration active de retenue et étant susceptibles d'être déplacées élastiquement vers leur configuration inactive dans laquelle elles échappent auxdites surfaces.

5. Ensemble selon la revendication 4, **caractérisé en ce que** les languettes élastiques (30, 32) sont orientées sensiblement axialement, sont solidaires du fourreau intérieur (18) et coopèrent dans leur configuration active avec des surfaces de retenue (22 ; 126A, 128A) formées sur la périphérie interne du fourreau de support (16 ; 116).

6. Ensemble selon la revendication 4 ou 5, **caractérisé en ce que** le piston (12 ; 112) de la seringue présente une tête d'actionnement (13 ; 113) apte (13B ; 113B) à coopérer avec les languettes (30, 32) pour solliciter ces dernières dans leur position inactive.

7. Ensemble selon les revendications 5 et 6, **caractérisé en ce que** les languettes élastiques (30, 32) présentent des extrémités libres (30A, 32A) qui s'étendent à travers des passages (26, 28 ; 126, 128) ménagés entre la collerette (20) du corps (10) de la seringue et la périphérie intérieure du fourreau de support (16 ; 116) et **en ce que** la tête d'actionnement (13 ; 113) du piston (12 ; 112) est apte (13B ; 113B) à coopérer avec les extrémités libres (30A, 32A) des languettes (30, 32) pour solliciter ces dernières dans leur position inactive.

8. Ensemble selon l'une quelconque des revendications 4 à 7, **caractérisé en ce que** la périphérie interne du fourreau de support (16) présente un épaulement (22) sur lequel sont formées à la fois la surface de butée pour la collerette (20) de la seringue et les surfaces de retenue pour les languettes élastiques (30, 32).

9. Ensemble selon l'une quelconque des revendications précédentes, **caractérisé en ce qu'**il comporte en outre des moyens de blocage (30, 32, 36 ; 136) aptes à bloquer le fourreau intérieur (18) dans sa position sortie de protection.

10. Ensemble selon la revendication 9, **caractérisé en ce que** les moyens de blocage comprennent une surface de blocage (36 ; 136) ménagée sur la périphérie interne du fourreau de support (16 ; 116) et au moins un élément de blocage (30, 32), solidaire du fourreau intérieur (18) et apte à coopérer avec cette surface en position sortie de protection du fourreau intérieur.

11. Ensemble selon les revendications 4 et 10, **caractérisé en ce que** les languettes élastiques (30, 32) constituent également des éléments de blocage aptes à coopérer avec la surface de blocage (36 ; 136) en position sortie de protection du fourreau intérieur (18).

12. Ensemble selon l'une quelconque des revendications précédentes, **caractérisé en ce que** les moyens de poussée comprennent un ressort (34) disposé autour du corps (10) de la seringue et prenant appui sur une première surface d'appui (20A ; 122A) appartenant à l'un des éléments constitués par le corps (10) de la seringue et par le fourreau de support (16 ; 116) et sur une deuxième surface d'appui (17B) appartenant au fourreau intérieur (18).

## Claims

1. An assembly comprising a safety support device for a syringe and a syringe having a body (10) presenting a collar (20), a piston (12), and a needle (14), the device comprising a support sheath (16; 116) for the syringe body and safety means comprising an inner sheath (18) capable of sliding relative to the support sheath between a waiting, retracted position in which the inner sheath (18) is substantially retracted inside the support sheath (16; 116) and is suitable for being placed around at least an end portion of the syringe body (10) carrying the needle (14), and a protective, extended position in which the inner sheath (18) projects beyond the support sheath to provide protection around the needle, the device further comprising retaining means (30, 32, 22; 126A, 128A) suitable for being activated to retain the inner sheath (18) in the waiting, retracted position inside the support sheath (16; 116) and for being deactivated to allow the inner sheath (18) to be extended under drive from thrust means (34),
the assembly being **characterised in that** it has holding means (22, 24; 122, 124) that are fixed with respect to the support sheath (16; 116) and that hold the syringe body (10) in fixed manner therein, said holding means comprising an abutment surface (22; 122) against which the collar (20) is held motionless by means forming a snap-fastening rib (24; 124), and **in that** the retaining means (30, 32, 22; 126A, 128A) are suitable for being deactivated as a function of the position of the piston (12) of the syringe, while the body (10) of the syringe remains fixed inside the support sheath (16; 116) by holding motionless the collar (20) of the syringe which is maintained by the holding means (22, 24; 122, 124).

2. An assembly according to claim 1, **characterised in that** the support sheath (116) has a rear end portion (146) and a main portion (147), said rear portion presenting holding means (122, 124) for the collar (20) of the body of the syringe (10) and a bearing surface (122A) for the thrust means (34).

3. An assembly according to claim 2, **characterised in that** the support sheath (116), the inner sheath (18), and the thrust means (34) form a unit in which the syringe body (10) can be inserted by axial thrust until the collar (20) of said body co-operates with said holding means (122, 124).

4. An assembly according to any one of claims 1 to 3, **characterised in that** the retaining means comprise resilient tongues (30, 32) and retaining surfaces (22; 126A, 128A), said tongues or said surfaces being integral with the inner sheath (18), the tongues (30, 32) being retained on the retaining surfaces (22; 126A, 128A) in their active, retaining configuration, and being suitable for being moved resiliently towards their inactive configuration in which they escape from said surfaces.

5. An assembly according to claim 4, **characterised in that** the resilient tongues (30, 32) are oriented substantially axially, are integral with the inner sheath (18), and co-operate in their active configuration with retaining surfaces (22; 126A, 128A) formed on the inner periphery of the support sheath (16; 116).

6. An assembly according to claim 4 or claim 5, **characterised in that** the piston (12; 112) of the syringe presents an actuation head (13; 113) suitable (13B; 113B) for co-operating with the tongues (30, 32) for urging them towards their inactive position.

7. An assembly according to claims 5 and 6, **characterised in that** the resilient tongues (30, 32) present free ends (30A, 32A) which extend through passages (26, 28; 126, 128) formed between the collar (20) of the body (10) of the syringe and the inner periphery of the support sheath (16; 116), and **in that** the actuation head (13; 113) of the piston (12; 112) is suitable (13B; 113B) for co-operating with the free ends (30A, 32A) of the tongues (30, 32) to urge the tongues towards their inactive position.

8. An assembly according to any one of claims 4 to 7, **characterised in that** the inner periphery of the support sheath (16) has a shoulder (22) on which there are formed both the abutment surface for the collar (20) of the syringe and the retaining surfaces for the resilient tongues (30, 32).

9. An assembly according to any one of the preceding claims, **characterised in that** it further comprises locking means (30, 32, 36; 136) suitable for locking the inner sheath (18) in its protective, extended position.

10. An assembly according to claim 9, **characterised in that** the locking means comprise a locking surface (36; 136) formed on the inner periphery of the support sheath (16; 116) and at least one locking element (30, 32) integral with the inner sheath (18) and suitable for co-operating with said surface when the inner sheath is in the protective, extended position.

11. An assembly according to claims 4 and 10, **characterised in that** the resilient tongues (30, 32) also constitute locking elements suitable for co-operating with the locking surface (36; 136) when the inner sheath (18) is in the protective, extended position.

12. An assembly according to any one of the preceding claims, **characterised in that** the thrust means comprise a spring (34) placed around the body (10) of the syringe and bearing against a first bearing surface (20A; 122A) belonging to one of the elements constituted by the syringe body (10) and by the support sheath (16; 116), and against a second bearing surface (17B) belonging to the inner sheath (18).

## Patentansprüche

1. Einheit aus einer Sicherheitsvorrichtung für eine Spritze und einer Spritze, umfassend einen Körper (10), der einen Kragen (20), einen Kolben (12) und eine Nadel (14) aufweist, wobei die Vorrichtung eine Stützhülle (16; 116) für den Spritzenkörper und Sicherheitsmittel umfaßt, die eine Innenhülle (18) aufweisen, die in Bezug auf die Stützhülle zwischen einer eingezogenen Warteposition, in der die Innenhülle (18) im wesentlichen in das Innere der Stützhülle (16, 116) eingezogen ist, wobei sie sich mindestens um einen Endabschnitt des Spritzenkörpers (10), der die Nadel (14) trägt, befinden kann, und einer ausgezogenen Spritzposition gleiten kann, in der die Innenhülle (18) über die Stützhülle hinaus ausfährt, um einen Schutz um die Nadel zu bilden, wobei die Vorrichtung ferner Haltemittel (30, 32, 22; 126A, 128A) umfaßt, die aktiviert werden können, um die Innenhülle (18) in der eingezogenen Warteposition in der Stützhülle (16; 116) zu halten, und die deaktiviert werden können, um das Ausfahren der Innenhülle (18) unter der Wirkung von Schubmitteln (34) zu gestatten,
**dadurch gekennzeichnet, daß** sie Verkeilungsmittel (22, 24; 122, 124) umfaßt, die mit der Stützhülle (16; 116) verbunden sind und den Spritzenkörper (10) fest in dieser letztgenannten halten, wobei die Verkeilungsmittel eine Anschlagfläche (22; 122) umfassen, an der der Kragen (20) durch Mittel, die eine Rastrippe (24, 124) bilden, festgestellt wird, und daß die Haltemittel (30, 32, 33; 126A, 128A) in Abhängigkeit von der Position des Kolbens (12) der Spritze deaktiviert werden können, während der Spritzenkörper (10) fest in der Stützhülle (16; 116) durch Feststellung des Kragens (20) der Spritze, der von den Verkeilungsmitteln (22, 24; 122, 124) gehalten wird, verbleibt.

2. Einheit nach Anspruch 1, **dadurch gekennzeichnet, daß** die Stützhülle (116) einen hinteren Endabschnitt (146) und einen Hauptabschnitt (147) umfaßt, wobei der hintere Abschnitt Mittel (122, 124) zur Verkeilung des Kragens (20) des Spritzenkörpers (10) und eine Stützfläche (122A) für die Schubmittel (34) umfaßt.

3. Einheit nach Anspruch 2, **dadurch gekennzeichnet, daß** die Stützhülle (116), die Innenhülle (18) und die Schubmittel (34) eine Gesamtheit bilden, in die der Spritzenkörper (10) durch einen Axialschub eingesetzt werden kann, bis der Kragen (20) des Körpers mit den Verkeilungsmitteln (122, 124) zusammenwirkt.

4. Einheit nach einem der Ansprüche 1 bis 3, **dadurch gekennzeichnet, daß** die Haltemittel elastische Zungen (30, 32) und Halteflächen (22; 126A, 128A) umfassen, wobei die Zungen oder Flächen mit der Innenhülle (18) verbunden sind, wobei die Zungen (30, 32) auf den Halteflächen (22; 126A, 128A) in ihrer aktiven Haltestellung gehalten werden und elastisch in ihre inaktive Stellung, in der sie aus den Flächen austreten, verschoben werden können.

5. Einheit nach Anspruch 4, **dadurch gekennzeichnet, daß** die elastischen Zungen (30, 32) im wesentlichen axial ausrichtet sind, mit der Innenhülle (18) verbunden sind und in ihrer aktiven Stellung mit Halteflächen (22; 126A, 128A) zusammenwirken, die am Innenumfang der Stützhülle (16; 116) ausgebildet sind.

6. Einheit nach Anspruch 4 oder 5, **dadurch gekennzeichnet, daß** der Kolben (12; 112) der Spritze einen Betätigungskopf (13; 113) aufweist, der mit den Zungen (30, 32) zusammenwirken kann (13B; 113B), um diese letztgenannten in ihre inaktive Stellung zu bringen.

7. Einheit nach den Ansprüchen 5 und 6, **dadurch gekennzeichnet, daß** die elastischen Zungen (30, 32) freie Enden (30A, 32A) aufwiesen, die sich durch Durchgänge (26, 28; 126, 128) erstrecken, die zwischen dem Kragen (20) des Spritzenkörpers (10) und dem Innenumfang der Stützhülle (16; 116) vorgesehen sind, und daß der Betätigungskopf (13; 113) des Kolbens (12; 112) mit den freien Enden (30A, 32A) der Zungen (30, 32) zusammenwirken kann (13B; 113B), um diese letztgenannten in ihre inaktive Stellung zu bringen.

8. Einheit nach einem der Ansprüche 4 bis 7, **dadurch gekennzeichnet, daß** der Innenumfang der Stützhülle (16) einen Absatz (22) aufweist, auf dem sowohl die Anschlagfläche für den Kragen (20) der Spritze als auch die Halteflächen für die elastischen Zungen (30, 32) ausgebildet sind.

9. Einheit nach einem der vorhergehenden Ansprüche, **dadurch gekennzeichnet, daß** sie ferner Feststellungsmittel (30, 32, 36; 136) umfaßt, die die Innenhülle (18) in ihrer ausgefahrenen Schutzposition feststellen können.

10. Einheit nach Anspruch 9, **dadurch gekennzeichnet, daß** die Feststellungsmittel eine Feststellungsfläche (36; 136), die am Innenumfang der Stützhülle (16; 116) vorgesehen ist, und mindestens ein Feststellungselement (30, 32) umfassen, das mit der Innenhülle (18) verbunden ist und mit dieser Fläche in der ausgefahrenen Schutzposition der Innenhülle zusammenwirken kann.

11. Einheit nach den Ansprüchen 4 und 10, **dadurch gekennzeichnet, daß** die elastischen Zungen (30, 32) auch Feststellungselemente darstellen, die mit der Feststellungsfläche (36; 136) in der ausgefahrenen Schutzposition der Innenhülle (18) zusammenwirken können.

12. Einheit nach einem der vorhergehenden Ansprüche, **dadurch gekennzeichnet, daß** die Schubmittel eine Feder (34) umfassen, die um den Spritzenkörper (10) angeordnet ist und auf einer ersten Stützfläche (20A; 122A), die einem der Elemente angehört, die von dem Spritzenkörper (10) und der Stützhülle (16; 116) gebildet sind, und auf einer zweiten Stützfläche (17B) aufliegt, die der Innenhülle (18) angehört.
